# EUROPEAN PATENT SPECIFICATION

(11) **EP 4 120 957 B1**
(45) Date of publication and mention of the grant of the patent: **15.07.2026**
(21) Application number: 21772280.0
(22) Date of filing: 16.03.2021
(51) Int. Cl.: A61B 17/15, A61B 34/20, A61B 34/30, A61B 34/10, A61F 2/30, A61B 17/56, A61B 90/00, A61F 2/46

(54) **REGISTRATION AND/OR TRACKING OF A PATIENT'S BONE EMPLOYING A PATIENT SPECIFIC BONE JIG**
REGISTRIERUNG UND/ODER VERFOLGUNG EINES PATIENTENKNOCHENS MIT EINER PATIENTENSPEZIFISCHEN KNOCHENEINSPANNVORRICHTUNG
ALIGNEMENT ET/OU SUIVI DE L'OS D'UN PATIENT À L'AIDE D'UN GABARIT OSSEUX SPÉCIFIQUE AU PATIENT

(30) Priority: 17.03.2020 US 202062990827 P
(43) Date of publication of application: 25.01.2023
(73) Proprietor: Icahn School of Medicine at Mount Sinai, New York, NY 10029 (US)
(72) Inventor: UNIS, Douglas B., New York, NY 10029 (US); SEXSON, Benjamin, Austin, TX 78744 (US); PATIL, Hrisheekesh, Austin, TX 78744 (US)
(74) Representative: Murgitroyd & Company
(86) International application number: PCT/US2021/022524
(87) International publication number: WO 2021/188512

(56) References cited:
- WO-A1-2013/188960
- WO-A1-2016/134168
- WO-A1-2019/148154
- WO-A1-2019/148154
- WO-A2-2014/045119
- US-A1- 2011 257 653
- US-A1- 2018 153 626
- US-A1- 2019 012 425
- US-A1- 2019 060 079

## Description

### CROSS REFERENCE TO RELATED APPLICATION

This application perfects and claims the priority benefit of U.S. Provisional Patent Application No. 62/990,827, filed March 17, 2020, entitled "Registration and/or Tracking of a Patient's Bone Employing a Patient Specific Bone Jig,".

### FIELD OF THE DISCLOSURE

The present disclosure relates generally to surgical methods, and more particularly to registration and/or tracking of a patient's bone employing a patient specific bone jig along with imaging and/or point sampling.

### BACKGROUND

Typically, computer assisted navigation procedures, pre-operative surgical imaging is frequently used to digitally capture an anatomical feature of interest such as a patient's bone. The process of registration and tracking is used, firstly, to provide the navigation system information about the actual starting position of the object of interest and secondly, to update the navigation system with movements of the tracked object over time. The registration process commonly involves a skilled user sampling clinically known anatomical landmarks with a tracked stylus. For example, with computer assisted navigation procedures for a hip, these points may be the apex of the greater or lesser trochanter. In other conventional registration processes, a tracked probe with fiducial markers is manually placed on the object of interest and sampled points are integrated into algorithms that infer the position of the object. By way of nonlimiting example, the fiducial markers may be visible to IR cameras, optical cameras or radar. The registration algorithms associate sets of data into a common coordinate system by minimizing the alignment error. The registration parameters define the transformation between the coordinate frame of the surface model and the coordinate frame of the robot. WO2014/045119A2, WO2019/148154A1, WO2013/188960A1, US2018/153626A1, US2011/257653A1 and WO2016/134168A1 disclose related art.

WO2014/045119A2 discloses systems and methods for creating a custom registration guide. In general, the methods may include receiving scan data of a patient's bone; creating instructions based on the scan data for creating a three-dimensional surface model of the patient's bone; and moving a plurality of moveable elements, coupled to the adjustable model, based on the instructions. The method may further include the steps of placing a malleable registration guide onto the adjustable model and shaping the registration guide to fit to the adjustable model. The method may further include the step of intraoperatively determining the location of a patient's bone using the custom registration guide. The step of determining the location of the patient's bone may include the steps of coupling the registration guide and fiducial markers to the patient's bone.

WO2019/148154A1 discloses a patient specific marker or template having one or more surfaces manufactured to fit a bone surface of a patient. The patient specific marker or template may optionally include color markings.

### SUMMARY

The invention is defined by appended claim 1.

Shortcomings of the prior art are overcome and additional advantages are provided through the provision, in one embodiment, of a computer implemented method which includes for example, obtaining, via one or more processors, three-dimensional data representing a patient's bone, obtaining, via the one or more processors, three-dimensional data representing at least portions of a patient specific bone jig, the patient specific bone jig having an inner surface portion matched to an outer surface portion of the patient's bone, obtaining, via the one or more processors, image data image data representing the at least portions of the patient specific bone jig, and generating, via the one or more processors, data representing a location and an orientation of the patient's bone based on the obtained image data, the obtained three-dimensional data representing the patient's bone, and the obtained three-dimensional data representing the at least portions of the patient specific bone jig.

In further examples, a computer implemented method includes for example, obtaining, via one or more processors, three-dimensional data representing a first object, obtaining, via the one or more processors, three-dimensional data representing at least portions of a second object, the second object having an inner surface portion matched to an outer surface portion of the first object, obtaining, via the one or more processors, image data representing the at least portions of the second object registered to the first object, and generating, via the one or more processors, data representing a location and/or an orientation of the second object based on the obtained image data, the obtained three-dimensional data representing the first object, and the obtained three-dimensional data representing at least portions of the second object.

In further examples, a computer implemented method includes for example, obtaining, via one or more processors, three-dimensional data representing the patient's bone, obtaining, via the one or more processors, three-dimensional data representing at least portions of a patient specific bone jig having predetermined spatial indicia, the patient specific bone jig having an inner surface portion matched to an outer surface portion of the patient's bone, obtaining, via the one or more processors, point sampling data representing the predetermined spatial indicia of the patient specific bone jig registered to the patient's bone, and generating data representing a location and an orientation of the patient's bone based on the obtained point sampling data, the obtained three-dimensional data representing the patient's bone, and the obtained three-dimensional data representing the at least portions of the patient specific bone jig having predetermined spatial indicia.

In further examples, a computer implemented method includes for example, obtaining, via one or more processors, three-dimensional data representing a first object, obtaining, via the one or more processors, three-dimensional data representing at least portions of a second object having predetermined spatial indicia, the second object having an inner surface portion matched to an outer surface portion of the first object, obtaining, via the one or more processors, point sampling data representing the predetermined spatial indicia of the second object registered to the first object, and generating, via the one or more processors, data representing a location and an orientation of the first object based on the obtained point sampling data, the obtained three-dimensional data representing the first object, and the obtained data three-dimensional data representing at least portions of the second object having the predetermined spatial indicia.

### BRIEF DESCRIPTION OF THE DRAWINGS

The subject matter which is regarded as the disclosure is particularly pointed out and distinctly claimed in the concluding portion of the specification. The disclosure, however, may best be understood by reference to the following detailed description of various embodiments and the accompanying drawings in which:
FIG. 1 is a flowchart of a process for registering and tracking a patient's bone, according to an embodiment of the present disclosure;
FIG. 2 is a perspective view of a patient specific bone jig attached to a proximal portion of a femur of a patient, according to an embodiment of the present disclosure;
FIG. 3 is a perspective view of a depth camera, according to an embodiment of the present disclosure;
FIG. 4 is a perspective view of the patient specific bone jig attached to the proximal portion of the femur of the patient of FIG. 2 along with a trackable fiducial array fixed to the patient's bone, according to an embodiment of the present disclosure;
FIG. 5 is a perspective view of an image shown in the foreground, obtained by the depth camera of FIG. 3, of the patient specific bone jig attached to the patient's bone shown in the background, according to an embodiment of the present disclosure;
FIG. 6 is a perspective view of a color filtered image shown in the foreground, obtained by the depth camera of FIG. 3, of the patient specific bone jig attached to the patient's bone shown in the background, according to an embodiment of the present disclosure;
FIG. 7 is a perspective view of a surface configuration, according to an embodiment of the present disclosure;
FIG. 8 is a perspective view of a surface configuration, according to an embodiment of the present disclosure;
FIG. 9 is a perspective view of a handheld depth camera, according to an embodiment of the present disclosure;
FIG. 10 is a perspective view of a robot with a tool and a plurality of cameras, according to an embodiment of the present disclosure;
FIG. 11 is a perspective view of a patient specific bone jig, according to an embodiment of the present disclosure;
FIG. 12 is a partial perspective view of the patient specific bone jig of FIG. 11, according to an embodiment of the present disclosure;
FIG. 13 is a perspective view of a patient specific bone jig, according to an embodiment of the present disclosure;
FIG. 14 is a first perspective view of a patient specific bone jig according to an embodiment of the present disclosure;
FIG. 15 is a second perspective view of the patient specific bone jig of FIG. 14, according to an embodiment of the present disclosure;
FIG. 16 is a perspective view of a patient specific bone jig with embedded fiducials, according to an embodiment of the present disclosure;
FIG. 17 is a block diagram of a system for use in registering and tracking a patient's bone, according to an embodiment of the present disclosure; and
FIG. 18 is a block diagram of a system for use in registering and tracking a patient's bone, according to an embodiment of the present disclosure.

### DETAILED DESCRIPTION

Generally stated, the present disclosure is directed to surgical methods, and more particularly to registration and tracking of a patient's bone employing a patient specific bone jig along with imaging and/or minimal point sampling of the patient's bone.

In this detailed description and the following claims, the words proximal, distal, anterior, posterior, medial, lateral, superior, and inferior are defined by their standard usage for indicating a particular part of a bone or implant according to the relative disposition of the natural bone or directional terms of reference. Positions or directions may be used herein with reference to anatomical structures or surfaces. For example, as the current devices and methods are described herein with reference to use with the bones of the hip, the bones of the hip may be used to describe the surfaces, positions, directions or orientations of the implant apparatus, implant installation apparatus, and surgical methods. Further, the devices and surgical methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the device and surgical methods, and the aspects, components, features and the like thereof, described and/or illustrated herein may be changed, varied, modified, reconfigured or otherwise altered for use or association with another side of the body for a same or similar purpose without departing from the scope of the disclosure. For example, the tools and methods, and the aspects, components, features and the like thereof, described herein with respect to a right femur may be mirrored so that they likewise function with a left femur and vice versa.

Registration is the process of matching actual anatomical structures such as an actual patient's bone with radiograph, fluoroscopy, and/or computed tomography (CT) scan data that has been entered into a computer. The present disclosure, in some embodiments, is directed generally to processes for reducing the time and increasing the accuracy of a registration process of aligning a pre-operative computer model to its real-time position or "pose" in the surgical field, and/or generally to processes for navigated tracking or updating the registered object's real-time position (generally once a known starting pose has been determined, although the pose can be captured at every sampled position). Mathematically, a transform may be determined to find the starting pose and this transform may then be applied to all of the real-time tracked positions. A benefit of the technique of the present disclosure may include elimination of the need for a manual point sampling registration method of the patient's bone itself while increasing the registering accuracy by sampling more points than may be possible in a comparable time. After registering the actual patient's bone with the computer model or data representing the patient's bone, further automated or robotic procedures may be performed including resection of the patient's bone.

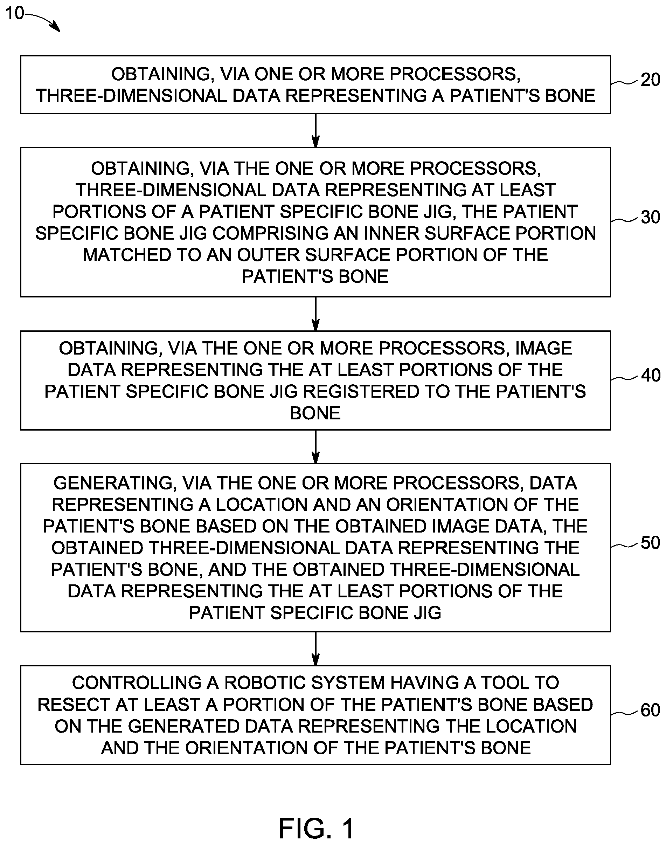

FIG. 1 illustrates a method 10 for use in registering and tracking a patient's bone, according to an embodiment of the present disclosure. For example, the method 10 may generally include in combination the use of an imaging system and the use of a patient specific bone jig.

In this illustrated embodiment, the method 10 may be operable to determine a location and an orientation of a patient's bone, and may include, for example, at 20 obtaining, via one or more processors, three-dimensional data representing a patient's bone, at 30 obtaining, via the one or more processors, three-dimensional data representing at least portions of a patient specific bone jig, the patient specific bone jig matched to a portion of the patient's bone, at 40 obtaining, via the one or more processors, image data representing the at least portions of the patient specific bone jig secured to the patient's bone, and at 50 generating, via the one or more processors, data representing the location and the orientation of the patient's bone based on the obtained image data, the obtained three-dimensional data representing the at least portions of the patient specific bone jig, and the obtained three-dimensional data representing the patient's bone. The method may further include at 60 controlling a robotic system having a tool to resect at least a portion of the patient's bone based on the generated data that represents the location and the orientation of the patient's bone.

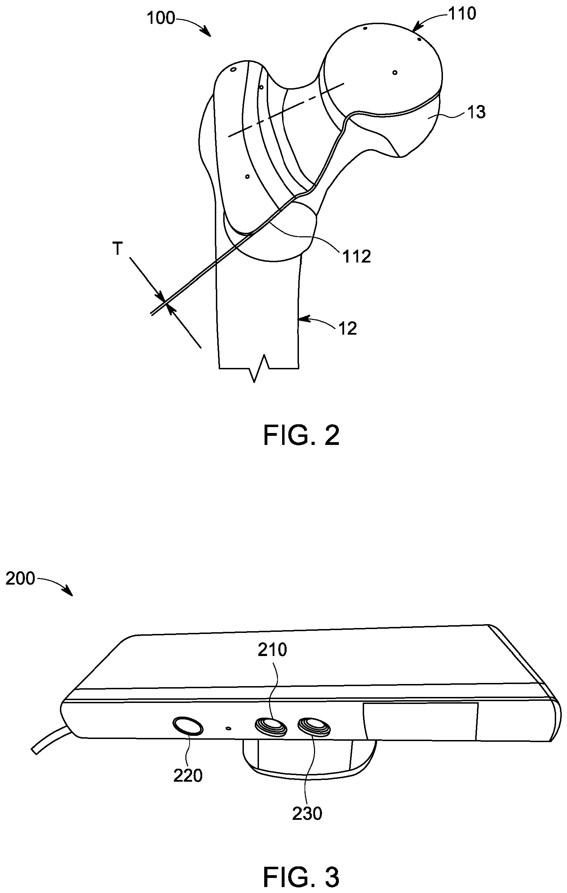

FIG. 2 illustrates a patient specific bone jig 100 secured to a patient's bone 12 such as a proximal portion of a femur, according to an embodiment of the present disclosure. In this illustrated embodiment, the patient specific bone jig 100 may include at least one or more features that allows coordinating or matching the patient specific bone jig 100 to the patient's bone 12. For example, the patient specific bone jig 100 may include a body 110 having a patient-specific inner surface contour 112 design to match a corresponding outer surface contour 13 of the patient's bone 12. As described in greater detail below, the patient specific bone jig 100 may be optimized for detection by a depth camera 200 (FIG. 3). As will be appreciated from the description below, knowledge of the model, shape, or portions of the patient specific bone jig 100 may allow quickly inferring the pose (e.g., location and/or orientation) data of the patient's bone 12 while obviating the need for complex computational image recognition algorithms as required in imaging just the patient's bone itself.

In some embodiments, one or more features or indicia of the patient specific bone jig 100 may be designed to optimize or aid the performance of a system that utilizes one or more depth cameras for registration and tracking of the patient's bone 12 in a clinical setting. For example, in some embodiments, the patient specific bone jig 100 may be based on features or indicia that include a color of the patient specific bone jig 100. In a surgical incision, there may be numerous colors present, for example skin tissue, fat tissue, muscle tissue, blood, cartilage, surgical retractors, sterilization drapes, etc., that have various colors. Furthermore, the lighting conditions present may be highly variable. The patient specific bone jig 100 may be designed with a non-organic, highly differentiated color such that it is likely to contrast with the surrounding scene. For example, the patient specific bone jig 100 may have a bright orange color or bright green color. In some embodiments, the patient specific bone jig may extend over an outer surface portion of the patient's bone and have a generally constant cross-sectional thickness T.

With reference to FIG. 3, a depth camera 200 may include an RGB (Red, Green, Blue) camera 210 and an infrared (IR) projector 220 and a near infrared (IR) sensor 230 such as a CMOS (Complementary Metal-Oxide-Semiconductor) sensor. The RGB camera 210 along with a processor and suitable programming may be operable to filter or mask colors within a specified RGB range. The RGB camera 210 can be used to filter or mask any objects, for example, that are not bright orange or bright green. Rather than relying on complex computational software to "identify" bone, the patient's bone can be identified via the patient specific bone jig. For example, the camera may be configured to mask everything but a bright green object (e.g., the bone jig). With the patient specific bone jig resting on and/or secured to the patient's bone in a known position (i.e., as it is patient specific), the underlying patient's bone position can be inferred, determined, or generated based on identifying the bright orange or green patient specific bone jig. In other embodiments, a depth camera may include a structured light projector and camera receiver, wherein a known pattern is projected onto a scene to calculate depth and surface information about the objects in the scene.

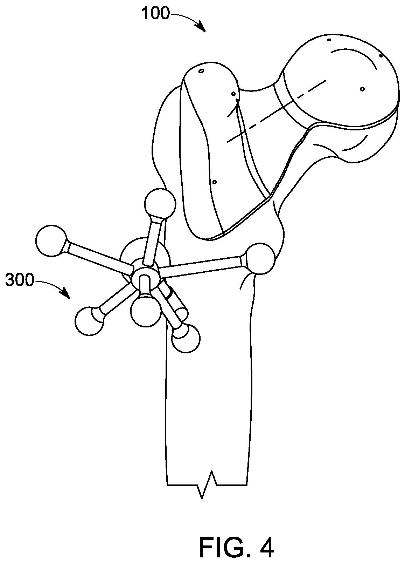

FIG. 4 illustrates the patient specific bone jig 100 with a fiducial array 300, according to an embodiment of the present disclosure. A registration may be performed utilizing a depth camera and a transform may be applied to track the patient specific bone jig 100 with the fiducial array 300 with conventional means compared to real-time object identification which is computationally demanding.

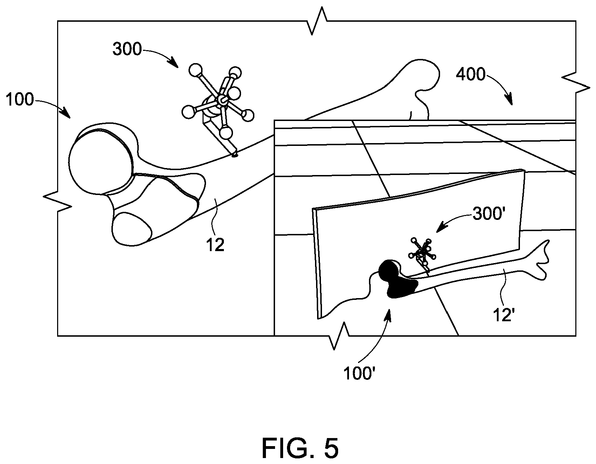

FIG. 5 illustrates a display image 400 (shown in the lower right in FIG. 5) representing the output of the scene from the perspective of the RGB camera 200 (FIG. 3) of the patient specific bone jig 100, the fiducial array 300, and the patient's bone 12 (shown in the upper left in FIG. 5). Data representing the patient specific bone jig 100, the fiducial array 300, and the patient's bone 12 may be obtained by the depth camera 200 (FIG. 3), and processed to form the image data representing a patient specific bone jig 100', the fiducial array 300', and the patient's bone 12' shown in the display image 400.

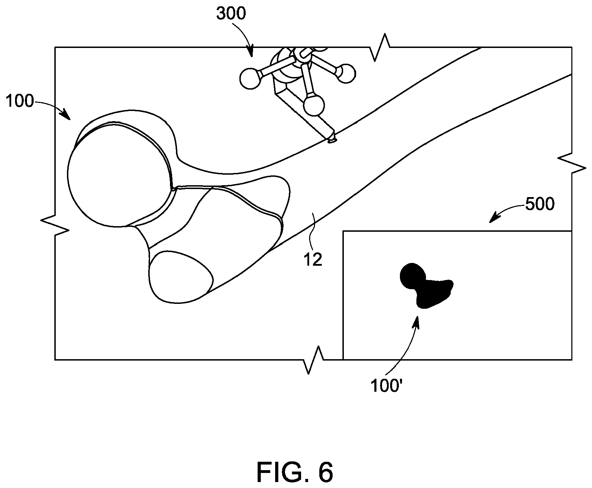

FIG. 6 illustrates a display image 500 (shown in the lower right in FIG. 6) of an output of the same scene as shown in FIG. 5 from the perspective of the RGB camera 200 of the patient specific bone jig 100, the fiducial array 300, and the patient's bone 12 (also shown in the upper left in FIG. 6) with a black color mask. In this illustrated embodiment of the display image 500, everything but the black object, i.e., the patient specific bone jig 100", is made to be visible. For example, all colors other than black may be made to be invisible in the display image 500.

As will be appreciated, using a color filter, a "noisy" scene may be simplified for further processing and computations. Accordingly, a colored patient specific bone jig 100 aids with object identification. The present technique may simplify and reduce the processing time necessary compared to the image 400 (FIG. 5) without a mask or filtering where all of the objects are visible, which increases the computational challenge to identify the patient's bone (e.g., especially with a limited exposure of time).

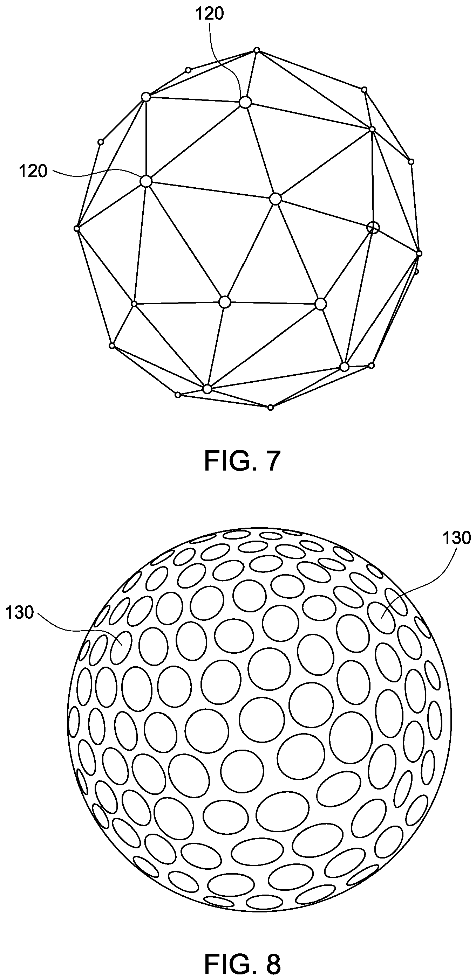

In some embodiments, a patient specific bone jig may utilize features or indicia such as a surface that is highly irregular with multiple sharp corners 120, such as a polygonal surface coating such as shown in FIG. 7. In some embodiments, a patient specific bone jig may also utilize features or indicia such as convex surface irregularities 130 to increase lighting contrast such as shown in FIG. 8. The different surface features or indicia may be used alone or in connection with the patient specific bone jig. The depth camera may employ a two-step process for inferring object position and/or orientation. For example, the depth cameral may utilize a fast feature detection algorithm followed by an ICP (Iterative Closest Point) optimization. A common method for accelerated feature identification may employ corner detection.

The patient specific bone jig may be used for registration, or for registration and tracking. For example, training an algorithm to identify the patient specific bone jig having known features or indicia such as a color and/or surface feature may simplify the challenge of utilizing a depth camera for registration and/or tracking. The depth camera may sample thousands of points at a time, which may increase the potential accuracy of the registration.

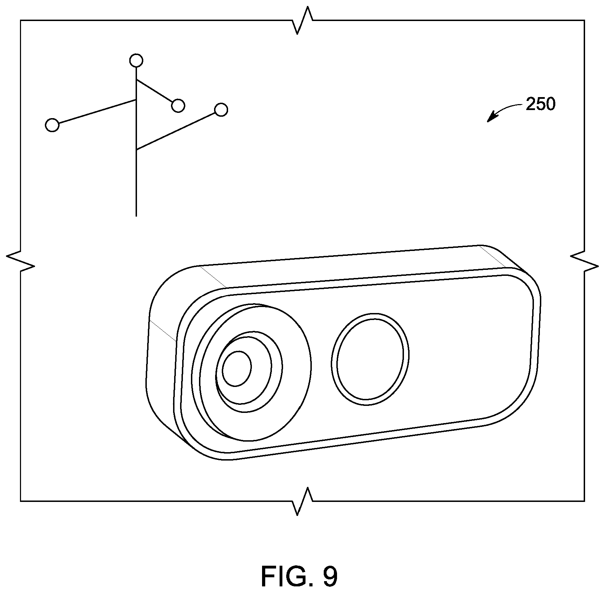

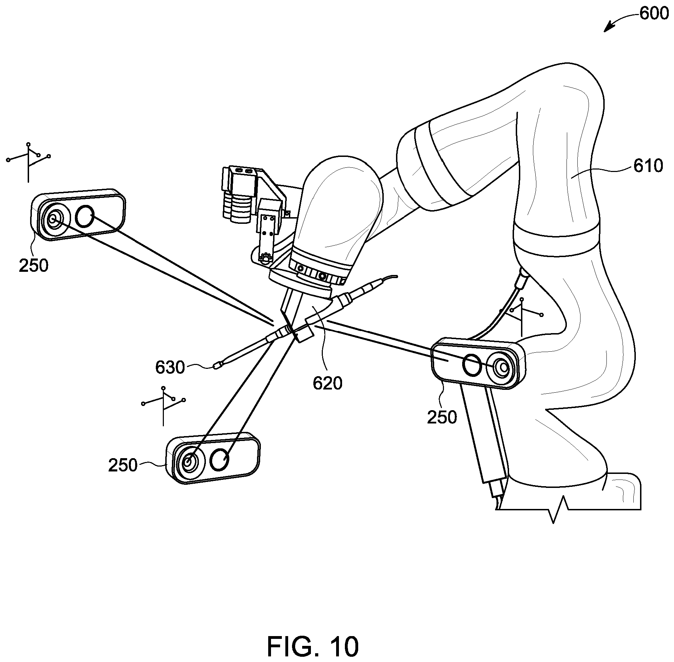

FIG. 9 illustrates a handheld depth camera 250 such as a RGBD camera that may be handheld and that may be tracked by a navigation system, e.g., fiducials or other devices that can be monitored by a navigation system such as radar. FIG. 10 illustrates a robot 600, a robotic arm 610, a robotic end-effector 620, a tool 630, and a plurality of depth cameras 250.

The position accuracy of robotic systems can be leveraged to calibrate the tool center point (TCP) to a known point on the robot, for example the flange. The end effector can be anodized or otherwise colored to allow for filtering with the RGBD camera. Known visible landmarks can be embedded at known positions on the robot and to which the TCP can be registered.

The RGBD camera can be used to identify the tool type that is engaged with the robot end-effector. For example, if the system calls for a 6 mm burr but a 9 mm burr is engaged, the RGBD camera can use fast feature detection algorithms to identify the object and return this information to the user. By way of nonlimiting example, if a spherical burr is engaged but a non-spherical burr is required, likewise the software algorithms can return this information to the user.

In another embodiment, the RGBD camera may be tracked such that the position of the camera at every sampled point is known. The TCP position can be inferred from multiple readings at multiple known positions (localization).

The handheld depth camera 250 may be used with a fast feature detection algorithm to accurately and quickly find the end-effector and tool tip pose and position. With the ability to accurately measure the special position of the tool tip, the robot can be moved to an assortment of positions (for example XYZ-4 point calibration) and quickly and accurately infer the tool offsets for the control algorithm. For example, the theoretical position may be compared to the actual position and infer a transform. Confirmation may be made of the proper tool (including diameter and tool type) and that the tool is fully engaged and properly seated within the end-effector of the robot for proper application.

Conventional methods for calibrating a robot may include touching reference parts, using distance sensors, and employing laser interferometry. A robot may also be calibrated using external sensors, such as camera systems, that can be attached to various robot locations to acquire the precise position of a reference object. The present disclosure using a camera capable of fast feature identification localizing position with localization algorithms may overcome problems associated with these conventional methods, which conventional methods are time-consuming and complicated.

Notably, the same color RGB filter, as previously described, can be used here. The end-effector, burr guide and tool for example may be colored or anodized to simplify the computational problem of identifying objects in the field. Notably, an advantage of this system is the ability to move the camera to more accurately localize object positions. In other setups, the sensors are often fixed and the robot is moved.

In some embodiments of the present disclosure, the depth camera registers the tool center point (TCP) to the robot flange or some known point on the robot. For example, industrial automation robots may include joint sensing (for example encoders) that return the exact position of each joint. The position of the flange in space can be known with high accuracy. The present disclosure proposes registering the TCP to the position of the flange in space. The depth camera would not need to be tracked by a navigation system to return to its position in space because the registration is to objects within its field of view (the tool tip to the flange). As a redundancy, the RGBD camera may then be tracked to confirm the TCP position is within an acceptable range of the expected position.

The depth camera may not only be used for pose estimation of tracked objects in the surgical field, but it can be used for calibration of the robot tool itself as well as confirmation that the proper cutting tool type is engaged (for example correct diameter and geometry burr) and properly seated in the cutting instrument. The robot desirably knows the exact location of the tool while it works. Each time a different tool is attached, the robot may need to be precisely re-calibrated. The TCP determines the exact working point of the tool in robot tool measurement.

Another technique of the present disclosure is directed to methods of utilizing a patient specific bone jig to improve the performance of conventional point sampling-based registration methods. Conventional point sampling methods are generally a two-step process including: 1) an approximation of the object's pose is determined by sampling points of anatomical importance, and 2) a more accurate position is determined by sampling more points and running an iterative closest point algorithm. The technique of the present disclosure may include patient specific bone jigs having indicia or features that allow readily coordinating the patient specific bone jig and the patient's bone with reduced point sampling.

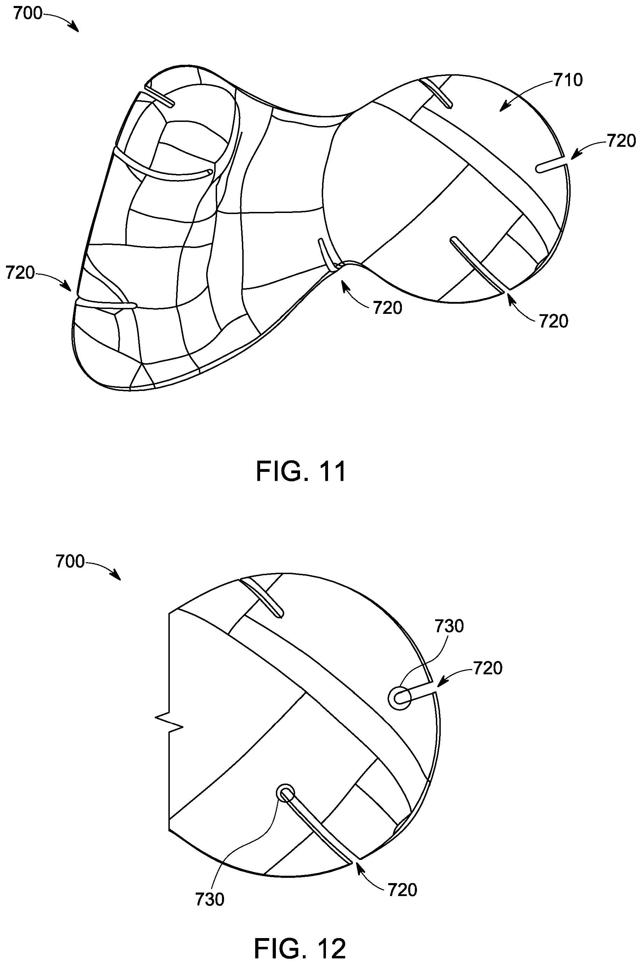

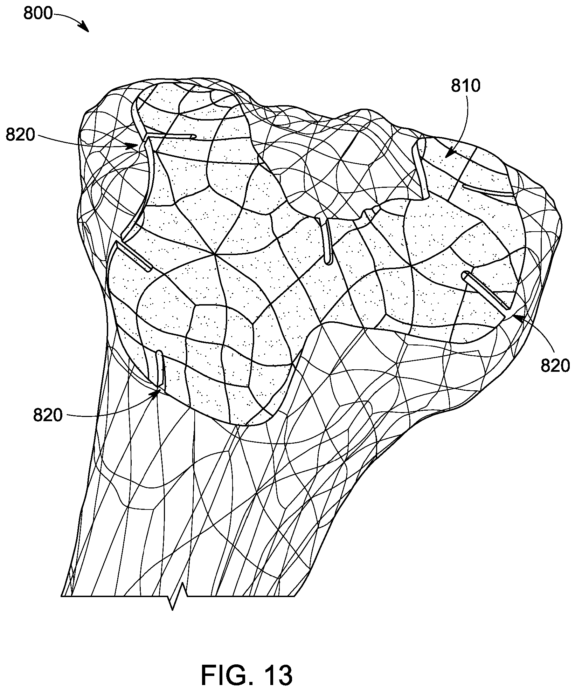

FIG. 11 illustrates a patient specific bone jig 700 having a body 710 with indicia or features such as channels 720 integrated into the body 710, according to an embodiment of the present disclosure. The locations of the channels 720 may be known or correlated relative to the body 710, which body 710 may also be configured for coordination with medical scans of the patient's bone. The channels 720 may allow for a reduced and more accurate sampling of points of interest with a registration probe. A registration probe can be placed at points of known positions on the patient specific bone jig, for example, at the extremity of a channel to sample points more accurately. With reference to FIG. 12, the registration probe tip may be placed at the distal end 730 of the channels 720 (in the circled areas in FIG. 12) to obtain more accurate position readings for the registration. FIG. 13 illustrates a patient specific knee bone jig 800 having a body 810 having channels 820. It will be appreciated that other channels, cutouts, or other passageways having different configurations may be suitably employed. For example, the point sampling may be performed to obtain an initial location and orientation of the patient specific bone jig registered and secured to the patient's bone. Thereafter, the imaging may be performed to track over time the location and orientation of the patient specific bone jig registered and secured to the patient's bone.

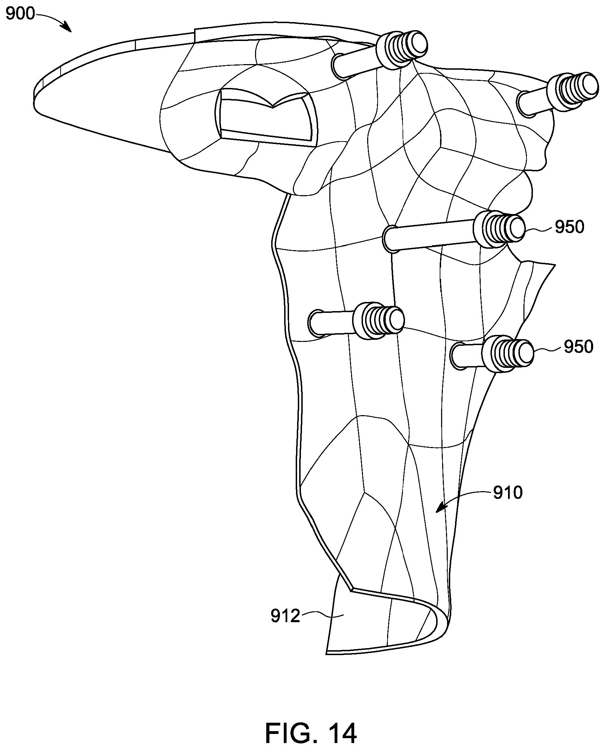

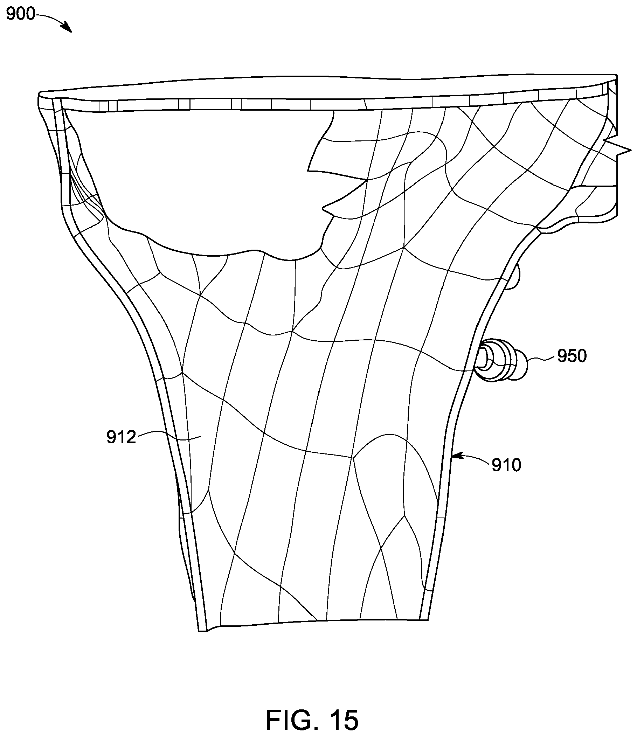

FIGS. 14 and 15 illustrate a patient specific bone jig 900 having a body 910 with indicia or features such as fiducial markers 950 integrated into or attached to the body 910, according to an embodiment of the present disclosure. In this illustrated embodiment, the patient specific bone jig 900 may include the body 910 having an inner surface 912 contoured to match the outer surface portion of the anatomy of the patient's bone and is generated from a pre-operative image or data such as a suitable medical scan. The inner surface of the patient specific bone jig 900 is contoured to match the patient anatomy for proper placement and alignment. The body 910 may include an exterior portion of the patient specific bone jig having one or more features or indicia onto which one or more tracked marker may be affixed. For example, the fiducial markers 950 may include threads and be threadably attached to the body 910 of the patient specific bone jig at certain locations and orientations. In other embodiments, the exterior portion could also include retroreflective tape or other markings or indicia that render it identifiable as a distinct object.

In some embodiments, the patient specific bone jig 900 may be employed in a registration process that does not rely on a tracked stylus to sample points as described below. For example, the data representing the patient specific bone jig with the fiducials is uploaded into a database that can be referenced by the navigation system. If the navigation system uses IR cameras, as an example, the array that matches the bone jig can be uploaded such that once identified by the navigation software, the patient's bone may be tracked. Similarly, a beacon may be attached to the jig such that the object may be tracked with radar. Once a user places the patient specific bone jig on the proper bone surface location, and the patent specific bone jig is properly identified and tracked by the navigation cameras, the user can sample the position of the patient specific bone jig and an initial pose estimate can be obtained.

A mathematical concept that is important with registration is the concept of the initial pose transform. The position of the tracked object (the patient's bone) will be inferred from the position of the patient specific bone jig. The navigation cameras may track the position of the patient specific bone jig, and not track the position of the patient's bone. The position of the patient's bone may be inferred, determined, and/or generated from the position of the patent specific bone jig, which is inferred, determined, and/or generated from the position of the tracked markers, for example the fiducial array.

To simplify these processes, attention may be made to the origin location for the patient's bone and the patient specific bone jig. When the pre-operative image data of the bone (for example the Dicom file from the CT scan) is generated, the data may be converted into a digital form that can be manipulated with computer aided design software. The digital files may have an origin location. The patient specific bone jig may be designed with the same origin frame as was used as the origin frame for the digital file of the patient's bone. When the navigation cameras track the patient specific bone jig, the origin point of the patient specific bone jig will correspond to the origin point of the patient's bone when the patient specific bone jig is properly placed on the exterior of the patient's bone.

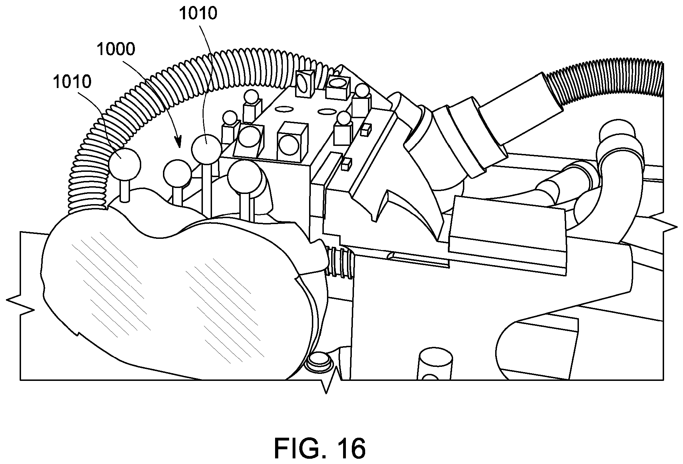

FIG. 16 illustrates a patient specific bone jig 1000 with embedded fiducials 1010 such as spherical IR arrays that may be used to track the patient specific bone jig. In the various embodiments, the patient specific bone jig may extend over the outer surface portion of the patient's bone and have a generally constant cross-sectional thickness. For example, the generally constant thickness may be between 1 millimeter and 10 millimeters. In the various embodiments, the patient specific bone jig may have a solid or single color such as a bright orange color, a bright green color, or the color black.

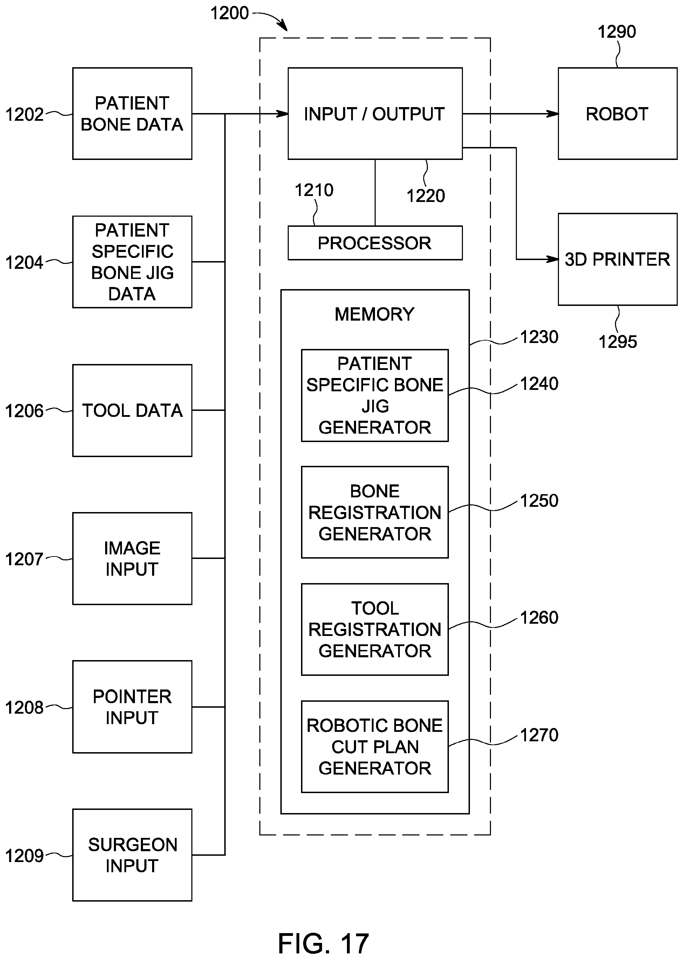

FIG. 17 illustrates a block diagram of a system 1200 for implementing, for example, the method 10 (FIG. 1), registration and/or tracking of a patient's bone, and/or fabricating a patient specific bone jig, according to an embodiment of the present disclosure. The system 1200 may generally include a processing unit or processor 1210, input/output devices 1220, and memory 1230.

For example, patient bone data 1202 such as three-dimensional data representing at least a portion of a patient's bone such as a proximal portion of a femur of the patient (block 20 in FIG. 1) may be obtained or inputted to the system 1200. The patient bone data 1202 may include three-dimensional data obtained by, for example, a Computed Tomography (CT) scan, a Computerized Axial Tomography (CAT) scan, a Magnetic Resonance Imaging (MRI) scan, or other suitable two-dimensional imaging or three-dimensional imaging or processing. Such data may be provided directly from an imaging machine or retrievable from a database of stored medical image data.

Patient specific bone jig data 1204 such as three-dimensional data or model representing at least a portion of a patient's specific bone jig (block 30 in FIG. 1) may be obtained or inputted to the system 1200. The patient specific bone jig data 1204 may include three-dimensional data previously generated, for example, based on scanning of the patient's specific bone jig or generated from the patient bone data 1202. For example, the system 1200 may include a patient specific bone jig generator 1240 operable for generating a patient specific bone jig based on the patient bone data 1202, and the patient specific bone jig generator 1240 may also be operable to control a 3D printer 1295 or other manufacturing process for fabricating the patient specific bone jig. The data for generating and fabrication of the patient specific bone jig may be the same or different from the patient specific bone jig data 1204. For example, the patient specific bone jig data 1204 may be limited to sufficient data representing portions of the patient specific bone jig allowing for use in the registration process.

Tool data 1206 such as three-dimensional data or a model representing at least a portion of a robotic tool, e.g., a working end such as a cutting end, may be obtained or inputted to the system 1200. The tool data 1202 may include three-dimensional data previously generated, for example, based on the scanning of the tool or data for fabricating the tool. For example, the tool data 1206 may be limited to sufficient data representing portions of the patient specific bone jig allowing for use in the robotic cut plan.

Image input 1207 such as image data may be obtained from an imager, camera, depth camera, RGB camera, IR sensor, or other suitable imager or scanning device representing at least a portion of a patient's specific bone jig (block 40 in FIG. 1), while the patient's specific bone jig is secured to the patient's bone. The image input 1207 may be inputted to the system 1200.

Pointer input 1208 such as pointer data can be obtained from a pointer or other suitable device for use in locating and orientating at least a portion of a patient's specific bone jig, while the patient's specific bone jig is secured to the patient's bone. The pointer data 1208 may be inputted to the system 1200.

Further input data may include surgeon input 1209 such as desired general data regarding location, orientation, tool, patient specific bone jig, or other data.

The processor 1210 may be a computer operating, for example, WINDOWS, OSX, UNIX or Linux operating system. In some embodiments, the processor 1210 may be a portable or handheld computing device. In other embodiments, the processing unit 1210 may be one or more operably connected processing units, computing devices, servers, linked or operating over one or more networks such as a global communications network, e.g., the Internet.

The memory 1230 may include various modules for processing the input data. For example, the memory 1230 may include the patient specific bone jig generator 1240, a bone registration generator 1250, a tool registration generator 1260, and a robotic bone cut plan generator 1270.

The patient specific bone jig generator 1240 may be operable to determine a configuration of a patient specific bone jig having, for example, an interior surface based on the patient specific bone data 1202, and coordinated or associated outer spatial indicia or fiducials. For example, the patient specific bone jig 1240 may determine the body of the patient specific bone jig having an interior surface that matches or corresponds to a portion of the patient's bone. In some embodiments, a 3D model of the patient specific bone jig may be used by the 3D printer 1295 or other manufacturing device known in the art for generating a patient specific bone jig. The 3D printer or manufacturing device may be operable to form patient specific bone jigs, as described above, as a one-piece, monolithic, or integral body, or formed from one or more components, and formed from a standard metallic material, polymeric material, or other suitable materials.

The bone registration generator 1250 may be operable to receive the patient bone data 1202, the patient specific bone jig data, and the imager input 1207 and/or the pointer input 1208 to determine the actual location and orientation of the patient's bone (block 50, FIG. 1) such as when the patient is disposed on an operating table. For example, using the patient bone data or bone model, suitable programing may be provided for locating and orienting the actual patient's bone based on the patient specific bone jig data and the observed actual patient specific bone jig (using imager or pointer) attached to the patient's bone.

The tool registration generator 1260 may be operable to receive the tool data 1206, and the imager input 1207, and/or the pointer input 1208 to determine the actual location and orientation of the tool such as when attached to the end of the robot with the patient disposed on an operating table and the patient specific bone jig secured to the patient's bone. For example, using the tool data or tool model, suitable programing may be provided for locating and orienting the actual tool based on the tool data and the observed (using imager or pointer) actual tool attached to the robot. The tool registration generator 1260 may be operable to also confirm or check that the proper tool is engaged for a specific procedure.

The robotic bone cut plan generator 1270 may be operable to determine data or instructions for operating a surgical robot 1290 or other automated devices to, for example, resect a patient's bone. In some embodiments, a 3D model of the resected bone, such as a resected proximal portion of the patient's femur or tibia may be uploaded to the surgical robot 1290 to allow the surgical robot to be operable to effect a bone cut plan to resize the proximal portion of the femur or tibia autonomously, or semi-autonomously to form, for example, a resection and/or form one or more cavities in the patient's bone. The data or instructions may be combined with data received by the surgical robot 1290 such as data from local cameras imagers or sensors. A suitable surgical robot may be an LBR iiwa Kuka robot manufactured by KUKA ROBOTICS Corporation of Shelby Township, Michigan, and may be operable with one or more bone saws, rasps, saws, drills, and/or other devices. The robotic bone cut plan generator 1270 may include various modules such as a resection surface or surface generator, a cavity or cavities generator, and an optimizing generator. The robotic bone cut plan generator 1270 may allow for a surgeon to indicate, for example, a resection plane or such plane may be automatically generated provided, e.g., by input from a surgeon, or based on or utilizing predetermined data. The robotic bone cut plan generator 1270 may include receiving initial inputs from a surgeon such as locations, widths, lengths, depths, or may be based on or utilizing predetermined data.

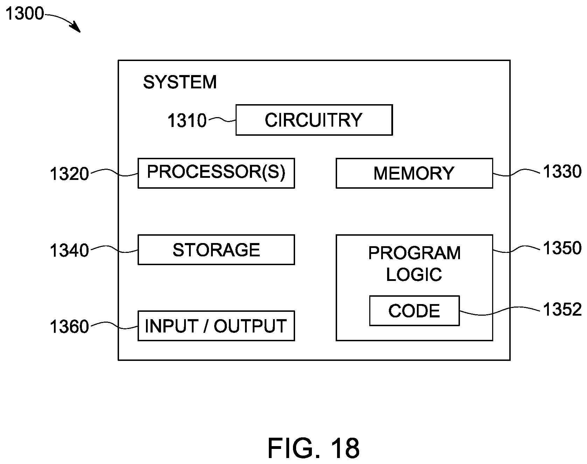

FIG. 18 illustrates a block diagram of another system 1300 for use in registration and/or tracking of a patient's bone employing a patient specific bone jig, which is part of the technical architecture of the embodiments of the present disclosure. System 1300 may include a circuitry 1310 that may in certain embodiments include a microprocessor 1320. The system 1300 may also include a memory 1330 (e.g., a volatile memory device), and storage 1340. The system 1300 may include program logic 1350 including code 1352 that may be loaded, via input/output 1360, into or stored in the memory 1330, the storage 1340, and/or circuitry 1310, and executed by the microprocessor 1320 and/or circuitry 1310. The various components may be operably coupled directly or indirectly via a system bus or may be coupled directly or indirectly to other data processing systems and components. The program logic 1350 may include the program code discussed above in this disclosure for use in forming, for example, a patient specific femoral stem of a femoral component for total hip replacement.

As will be appreciated by one skilled in the art, aspects of the technique may be embodied as a system, method, or computer program product. Accordingly, aspects of the technique may take the form of an entirely hardware embodiment, an entirely software embodiment (including firmware, resident software, micro-code, etc.), or an embodiment combining software and hardware aspects that may all generally be referred to herein as a "circuit," "module," or "system."

It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. Each block in the flowchart or block diagrams may represent a module, segment, or portion of code, which includes one or more executable instructions for implementing the specified logical function(s).

These computer program instructions, also referred to as software and/or program code, may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks. For example, in a particular arrangement, a desktop or workstation computer may be employed using a commercially available operating system, e.g., Windows^{®}, OSX^{®}, UNIX or Linux based implementation.

The computer readable storage medium 1340 may be, for example, but not limited to, an electronic, magnetic, optical, electromagnetic, infrared or semiconductor system, apparatus, or device, or any suitable combination of the foregoing. The storage 1340 may include an internal storage device, an attached storage device and/or a network accessible storage device. More specific examples (a non-exhaustive list) of the computer readable storage medium include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain or store a program for use by or in connection with an instruction execution system, apparatus, or device.

Computer program code for carrying out operations for aspects of the present technique may be written in any combination of one or more programming languages, including an object oriented programming language, such as Java, Smalltalk, C++ or the like, and conventional procedural programming languages, such as the "C" programming language, PHP, ASP, assembler or similar programming languages, as well as functional programming languages and languages for technical computing. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). Furthermore, more than one computer can be used for implementing the program code, including, but not limited to, one or more resources in a cloud computing environment.

The Input/Output or I/O devices 1360 (including, but not limited to, keyboards, displays, pointing devices, DASD, tape, CDs, DVDs, thumb drives and other memory media, etc.) can be coupled to the system either directly or through intervening I/O controllers. Network adapters may also be coupled to the system to enable the data processing system to become coupled to other data processing systems or remote printers or storage devices through intervening private or public networks. Modems, cable modems, and Ethernet cards are just a few of the available types of network adapters.

Data relating to a patient, e.g., the patient's pelvis and hip, may be created by, or accessed from, a medical sensor device. For example, previous medical scans of an extremity, such as those obtained from a computerized axial tomography (CAT or CT) or magnetic resonance imaging (MRI) scan may be stored in a medical record storage apparatus, in storage 1340, or accessed by system 1300. Such patient data may include other data for a given patient (e.g., bone density, type, length, medical conditions etc.). By way of a non-limiting example, the patient data may include a scan data set containing a series of two-dimensional images obtained from the scanning device (e.g., CT scan slices). As such, the scan data set is a 3D dimensional representation of the scan data.

It will be appreciated that the technique of the present disclosure may overcome the time and processing constraints of current state-of-the-art registration and tracking which employs manually sampling a large number of points of interest. In particular, the technique of the present disclosure may overcome the disadvantage of current registration methods that include the needed time required to manually sample a large number of points to achieve a high accuracy registration and the need for the registration to be performed by a skilled user.

For example, with the use of a camera, the present technique may allow for thousands of points to be "sampled" in a very short time, increasing registration accuracy and reducing time. The registration can also be performed by a less skilled user.

From the present description, it will be appreciated that the technique of the present disclosure may provide methods for optimizing the scene and a target to enhance the utility of depth cameras in the surgical field. To utilize a depth camera for registration and tracking in the surgical field, the camera may be operable to 1) identify the clinically relevant object to track, and 2) determine the pose or spatial position and orientation of the object.

The present technique employing patient specific bone jigs with imaging and/or minimal point sampling overcomes problems associated with current registration and tracking. To algorithmically identify anatomical structures is a non-trivial computational problem that may involve machine learning or complex geometric modelling techniques. Determining the pose or spatial position and orientation of an object is also challenging. In clinical practice exposures are often small and the features of a surgical site can be highly variable. Algorithmic feature detection with which to determine spatial orientation can prove a challenge. The availability of data on which to "train" or validate these algorithms is another major impediment to development. Conventional feature detection algorithms on which depth cameras rely struggle correlating to models with small exposures with limited surface anomalies (smooth) in "noisy" environments (cartilage, blood, surgical tools and other soft tissues). Data sets of real-world procedures to train the algorithms are difficult to obtain. Furthermore, unlike spinal anatomy, identifying key anatomical landmarks with knees and hips in limited exposure can be quite challenging.

For example, the technique of the present disclosure may overcome the problems of conventional registration where a user sampling clinically known anatomical landmarks is subjective and prone to error. The present technique reduces the need in conventional registrations of having to sample a high number of points to increase accuracy, which increased sampling increases surgery time. Additionally, because the sampling of points requires a highly skilled user, such as a surgeon, it limits the ability of lower skilled users to support the task. Generating high accuracy registrations in a timely manner continues to be a challenge in the industry.

As may be recognized by those skilled in the art based on the teachings herein, numerous changes and modifications may be made to the above-described and other embodiments of the present invention without departing from the scope of the invention. The jigs, programming, other components of the systems, devices and/or apparatus as disclosed in the specification, including the accompanying abstract and drawings, may be replaced by alternative component(s) or feature(s), such as those disclosed in another embodiment, which serve the same, equivalent or similar purpose as known by those skilled in the art to achieve the same, equivalent or similar results by such alternative component(s) or feature(s) to provide a similar function for the intended purpose. In addition, the devices and apparatus may include more or fewer components or features than the embodiments as described and illustrated herein. Accordingly, this detailed description of the currently-preferred embodiments is to be taken as illustrative, as opposed to limiting the disclosure.

The technique of the present disclosure may be employed with the one or more components described in U.S. provisional patent application no. 62/854,648, filed May 30, 2019, by Sexson et al., entitled "Robot Mounted Camera Registration and Tracking System for Orthopedic and Neurological Surgery". For example, the patient specific bone jig may be employed as the marker.

The technique of the present disclosure may be employed with the one or more components described in U.S. provisional patent application no. 62/850,050, filed May 20, 2019, by Sexson et al., entitled "A System And Method For Interaction And Definition Of Tool Pathways For A Robotic Cutting Tool".

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the architectural and operational embodiments described herein are exemplary of a plurality of possible arrangements to provide the same general features, characteristics, and general apparatus operation. Modifications and alterations will occur to others upon a reading and understanding of the preceding detailed description.

## Claims

1. A computer program product comprising instructions which, when the program is executed by a computer, cause the computer to carry out a method, the method comprising:
obtaining (20), via one or more processors, three-dimensional data representing a patient's bone (12);
obtaining (30), via the one or more processors, three-dimensional data representing at least portions of a patient specific bone jig (100), the patient specific bone jig comprising an inner surface portion (112) matched to an outer surface portion (13) of the patient's bone;
obtaining (40), via the one or more processors, image data representing the at least portions of the patient specific bone jig secured to the patient's bone; and
generating (50), via the one or more processors, data representing a location and an orientation of the patient's bone based on the obtained image data, the obtained three-dimensional data representing the patient's bone, and the obtained three-dimensional data representing at least portions of the patient specific bone jig; and
wherein the patient specific bone jig (100) comprises a first color, and the obtaining image data comprises using an RGB camera, and filtering, via the one or more processors, colors other than the first color in the image, and wherein the generating data representing the location and the orientation of the patient's bone (12) is based on the filtered image.

2. The computer program product of claim 1, wherein the patient specific bone jig (100) comprises outwardly-extending fiducials, and the generating data representing the location and the orientation of the patient's bone (12) is based on the imaged outwardly extending fiducials in the obtained image data.

3. The computer program product of claim 1, wherein the patient specific bone jig (100) comprises a plurality of indicia, and the generating data representing the location and the orientation of the patient's bone (12) is based on imaged indicia in the obtained image data, wherein the plurality of indicia comprises a plurality of channels (720) and the generating data representing the location and the orientation of the patient's bone is based on the imaged plurality of channels in the obtained image data, and wherein the plurality of channels extend through the thickness of the patient specific bone jig.

4. The computer program product of claim 1, wherein the patient specific bone jig comprises a plurality of indicia and the plurality of indicia comprises corners, and the generating data representing the location and the orientation of the patient's bone (12) is based on imaged corners in the obtained image data.

5. The computer program product of claim 1, wherein the patient specific bone jig comprises a plurality of indicia and the plurality of indicia comprises a plurality of convex surface irregularities (130), and the generating data representing the location and the orientation of the patient's bone (12) is based on imaged convex surface irregularities in the obtained image data.

6. The computer program product of claim 1, the method further comprising:
tracking, via the one or more processors, the location and orientation of the patient's bone (12) based on the generated data over time.

7. The computer program product of claim 1, wherein the patient specific bone jig (100) extends over the outer surface portion of the patient's bone (12) and comprises a generally constant cross-sectional thickness, wherein the generally constant thickness is between 1 millimeter and 10 millimeters.

8. The computer program product of claim 1, the method further comprising controlling, via the one or more processors, a 3D printer (1295) or other manufacturing process for fabricating the patient specific bone jig (100).

9. The computer program product of claim 8, the method further comprising:
generating, via the one or more processors, the three-dimensional data representing the patient specific bone jig (100) based on the obtained three-dimensional data representing the patient's bone (12).

10. The computer program product of claim 1, wherein the obtaining, via the one or more processors, the three-dimensional data representing the patient's bone (12) comprises obtaining, via the one or more processors, a scan from a medical scanning device of the patient's bone.

11. The computer program product of claim 10, wherein the scan comprises a CAT scan and/or an MRI scan.

12. The computer program product of claim 1, wherein the obtaining, via the one or more processors, the image data comprises obtaining, via the one or more processors, an image or scan from a handheld imager.

## Patentansprüche

1. Ein Computerprogrammprodukt, das Anweisungen beinhaltet, die, wenn das Programm von einem Computer ausgeführt wird, den Computer veranlassen, ein Verfahren zu vollziehen, wobei das Verfahren Folgendes beinhaltet:
Erhalten (20), über einen oder mehrere Prozessoren, von dreidimensionalen Daten, die einen Knochen (12) eines Patienten darstellen;
Erhalten (30), über den einen oder die mehreren Prozessoren, von dreidimensionalen Daten, die mindestens Abschnitte einer patientenspezifischen Knochenschablone (100) darstellen, wobei die patientenspezifische Knochenschablone einen Innenoberflächenabschnitt (112) beinhaltet, der an einen Außenoberflächenabschnitt (13) des Knochens des Patienten angepasst ist;
Erhalten (40), über den einen oder die mehreren Prozessoren, von Bilddaten, die die mindestens Abschnitte der patientenspezifischen Knochenschablone, die an dem Knochen des Patienten befestigt ist, darstellen; und
Erzeugen (50), über den einen oder die mehreren Prozessoren, von Daten, die eine Lage und eine Ausrichtung des Knochens des Patienten darstellen, basierend auf den erhaltenen Bilddaten, den erhaltenen dreidimensionalen Daten, die den Knochen des Patienten darstellen, und den erhaltenen dreidimensionalen Daten, die mindestens Abschnitte der patientenspezifischen Knochenschablone darstellen; und
wobei die patientenspezifische Knochenschablone (100) eine erste Farbe beinhaltet und das Erhalten von Bilddaten das Verwenden einer RGB-Kamera und das Filtern, über den einen oder die mehreren Prozessoren, von anderen Farben als der ersten Farbe in dem Bild beinhaltet und wobei das Erzeugen von Daten, die die Lage und die Ausrichtung des Knochens (12) des Patienten darstellen, auf dem gefilterten Bild basiert.

2. Computerprogrammprodukt gemäß Anspruch 1, wobei die patientenspezifische Knochenschablone (100) sich nach außen erstreckende Referenzmarkierungen beinhaltet und das Erzeugen von Daten, die die Lage und die Ausrichtung des Knochens (12) des Patienten darstellen, auf den abgebildeten sich nach außen erstreckenden Referenzmarkierungen in den erhaltenen Bilddaten basiert.

3. Computerprogrammprodukt gemäß Anspruch 1, wobei die patientenspezifische Knochenschablone (100) eine Vielzahl von Kennzeichen beinhaltet und das Erzeugen von Daten, die die Lage und die Ausrichtung des Knochens (12) des Patienten darstellen, auf abgebildeten Kennzeichen in den erhaltenen Bilddaten basiert, wobei die Vielzahl von Kennzeichen eine Vielzahl von Kanälen (720) beinhaltet und das Erzeugen von Daten, die die Lage und die Ausrichtung des Knochens des Patienten darstellen, auf der abgebildeten Vielzahl von Kanälen in den erhaltenen Bilddaten basiert und wobei sich die Vielzahl von Kanälen durch die Dicke der patientenspezifischen Knochenschablone erstreckt.

4. Computerprogrammprodukt gemäß Anspruch 1, wobei die patientenspezifische Knochenschablone eine Vielzahl von Kennzeichen beinhaltet und die Vielzahl von Kennzeichen Ecken beinhaltet und das Erzeugen von Daten, die die Lage und die Ausrichtung des Knochens (12) des Patienten darstellen, auf abgebildeten Ecken in den erhaltenen Bilddaten basiert.

5. Computerprogrammprodukt gemäß Anspruch 1, wobei die patientenspezifische Knochenschablone eine Vielzahl von Kennzeichen beinhaltet und die Vielzahl von Kennzeichen eine Vielzahl von konvexen Oberflächenunregelmäßigkeiten (130) beinhaltet und das Erzeugen von Daten, die die Lage und die Ausrichtung des Knochens (12) des Patienten darstellen, auf abgebildeten konvexen Oberflächenunregelmäßigkeiten in den erhaltenen Bilddaten basiert.

6. Computerprogrammprodukt gemäß Anspruch 1, wobei das Verfahren ferner Folgendes beinhaltet:
Verfolgen, über den einen oder die mehreren Prozessoren, der Lage und Ausrichtung des Knochens (12) des Patienten basierend auf den erzeugten Daten im Zeitverlauf.

7. Computerprogrammprodukt gemäß Anspruch 1, wobei sich die patientenspezifische Knochenschablone (100) über den Außenoberflächenabschnitt des Knochens (12) des Patienten erstreckt und eine im Allgemeinen konstante Querschnittsdicke beinhaltet, wobei die im Allgemeinen konstante Dicke zwischen 1 Millimeter und 10 Millimeter beträgt.

8. Computerprogrammprodukt gemäß Anspruch 1, wobei das Verfahren ferner Folgendes beinhaltet:
Steuern, über den einen oder die mehreren Prozessoren, eines 3D-Druckers (1295) oder eines anderen Herstellungsprozesses zum Fertigen der patientenspezifischen Knochenschablone (100).

9. Computerprogrammprodukt gemäß Anspruch 8, wobei das Verfahren ferner Folgendes beinhaltet:
Erzeugen, über den einen oder die mehreren Prozessoren, der dreidimensionalen Daten, die die patientenspezifische Knochenschablone (100) darstellen, basierend auf den erhaltenen dreidimensionalen Daten, die den Knochen (12) des Patienten darstellen.

10. Computerprogrammprodukt gemäß Anspruch 1, wobei das Erhalten, über den einen oder die mehreren Prozessoren, der dreidimensionalen Daten, die den Knochen (12) des Patienten darstellen, das Erhalten, über den einen oder die mehreren Prozessoren, eines Scans von einer medizinischen Scanvorrichtung des Knochens des Patienten beinhaltet.

11. Computerprogrammprodukt gemäß Anspruch 10, wobei der Scan einen CAT-Scan und/oder einen MRT-Scan beinhaltet.

12. Computerprogrammprodukt gemäß Anspruch 1, wobei das Erhalten, über den einen oder die mehreren Prozessoren, der Bilddaten das Erhalten, über den einen oder die mehreren Prozessoren, eines Bildes oder Scans von einem tragbaren Bildgeber beinhaltet.

## Revendications

1. Un produit programme d'ordinateur comprenant des instructions qui, lorsque le programme est exécuté par un ordinateur, amènent l'ordinateur à mettre en œuvre une méthode, la méthode comprenant :
l'obtention (20), par l'intermédiaire d'un ou de plusieurs processeurs, de données tridimensionnelles représentant un os d'un patient (12) ;
l'obtention (30), par l'intermédiaire des un ou plusieurs processeurs, de données tridimensionnelles représentant au moins des portions d'un gabarit osseux spécifique au patient (100), le gabarit osseux spécifique au patient comprenant une portion de surface interne (112) coïncidant avec une portion de surface externe (13) de l'os du patient ;
l'obtention (40), par l'intermédiaire des un ou plusieurs processeurs, de données d'image représentant les au moins portions du gabarit osseux spécifique au patient assujetti à l'os du patient ; et
la génération (50), par l'intermédiaire des un ou plusieurs processeurs, de données représentant un emplacement et une orientation de l'os du patient sur la base des données d'image obtenues, des données tridimensionnelles obtenues représentant l'os du patient, et des données tridimensionnelles obtenues représentant au moins des portions du gabarit osseux spécifique au patient ; et
où le gabarit osseux spécifique au patient (100) comprend une première couleur, et l'obtention de données d'image comprend l'utilisation d'une caméra RVB, et le filtrage, par l'intermédiaire des un ou plusieurs processeurs, de couleurs autres que la première couleur dans l'image, et où la génération de données représentant l'emplacement et l'orientation de l'os du patient (12) est basée sur l'image filtrée.

2. Le produit programme d'ordinateur de la revendication 1, où le gabarit osseux spécifique au patient (100) comprend des repères s'étendant vers l'extérieur, et la génération de données représentant l'emplacement et l'orientation de l'os du patient (12) est basée sur les repères imagés s'étendant vers l'extérieur dans les données d'image obtenues.

3. Le produit programme d'ordinateur de la revendication 1, où le gabarit osseux spécifique au patient (100) comprend une pluralité de marques, et la génération de données représentant l'emplacement et l'orientation de l'os du patient (12) est basée sur des marques imagées dans les données d'image obtenues, où la pluralité de marques comprend une pluralité de canaux (720) et la génération de données représentant l'emplacement et l'orientation de l'os du patient est basée sur la pluralité de canaux imagés dans les données d'image obtenues, et où la pluralité de canaux s'étend à travers l'épaisseur du gabarit osseux spécifique au patient.

4. Le produit programme d'ordinateur de la revendication 1, où le gabarit osseux spécifique au patient comprend une pluralité de marques et la pluralité de marques comprend des coins, et la génération de données représentant l'emplacement et l'orientation de l'os du patient (12) est basée sur des coins imagés dans les données d'image obtenues.

5. Le produit programme d'ordinateur de la revendication 1, où le gabarit osseux spécifique au patient comprend une pluralité de marques et la pluralité de marques comprend une pluralité d'irrégularités de surface convexes (130), et la génération de données représentant l'emplacement et l'orientation de l'os du patient (12) est basée sur des irrégularités de surface convexes imagées dans les données d'image obtenues.

6. Le produit programme d'ordinateur de la revendication 1, la méthode comprenant en outre :
le suivi, par l'intermédiaire des un ou plusieurs processeurs, de l'emplacement et de l'orientation de l'os du patient (12) sur la base des données générées au fil du temps.

7. Le produit programme d'ordinateur de la revendication 1, où le gabarit osseux spécifique au patient (100) s'étend par-dessus la portion de surface externe de l'os du patient (12) et comprend une épaisseur en coupe transversale généralement constante, où l'épaisseur généralement constante est comprise entre 1 millimètre et 10 millimètres.

8. Le produit programme d'ordinateur de la revendication 1, la méthode comprenant en outre :
la commande, par l'intermédiaire des un ou plusieurs processeurs, d'une imprimante 3D (1295) ou d'un autre procédé de fabrication pour réaliser le gabarit osseux spécifique au patient (100).

9. Le produit programme d'ordinateur de la revendication 8, la méthode comprenant en outre :
la génération, par l'intermédiaire des un ou plusieurs processeurs, des données tridimensionnelles représentant le gabarit osseux spécifique au patient (100) sur la base des données tridimensionnelles obtenues représentant l'os du patient (12).

10. Le produit programme d'ordinateur de la revendication 1, où l'obtention, par l'intermédiaire des un ou plusieurs processeurs, des données tridimensionnelles représentant l'os du patient (12) comprend l'obtention, par l'intermédiaire des un ou plusieurs processeurs, d'un scan, à partir d'un dispositif scanner médical, de l'os du patient.

11. Le produit programme d'ordinateur de la revendication 10, où le scan comprend un scan TDM et/ou un scan IRM.

12. Le produit programme d'ordinateur de la revendication 1, où l'obtention, par l'intermédiaire des un ou plusieurs processeurs, des données d'image comprend l'obtention, par l'intermédiaire des un ou plusieurs processeurs, d'une image ou d'un scan à partir d'un imageur portatif.
